# EUROPÄISCHE PATENTSCHRIFT

(11) **EP 0 772 768 B1**
(45) Veröffentlichungstag und Bekanntmachung des Hinweises auf die Patenterteilung: **07.10.1998**
(21) Anmeldenummer: 95928474.6
(22) Anmeldetag: 27.07.1995
(51) Int. Cl.: G01N 21/47, A61B 5/00

(54) **APPARAT UND METHODE ZUR OPTISCHEN CHARAKTERISIERUNG VON STRUKTUR UND ZUSAMMENSETZUNG EINER STREUENDEN PROBE**
APPARATUS AND PROCESS FOR OPTICAL CHARACTERISATION OF STRUCTURE AND COMPOSITION OF A SCATTERING SAMPLE
PROCEDE ET APPAREIL PERMETTANT DE CARACTERISER DE MANIERE OPTIQUE LA STRUCTURE ET LA COMPOSITION D'UN ECHANTILLON DISPERSIF

(30) Priorität: 30.07.1994 DE 4427101
(43) Veröffentlichungstag der Anmeldung: 14.05.1997
(73) Patentinhaber: BOEHRINGER MANNHEIM GMBH, 68298 Mannheim (DE)
(72) Erfinder: HEIN, Heinz-Michael, D-64342 Seeheim-Jugenheim (DE); BOECKER, Dirk, D-69115 Heidelberg (DE); ESSENPREIS, Matthias, D-82131 Gauting (DE)
(86) Internationale Anmeldenummer: EP9502967
(87) Internationale Veröffentlichungsnummer: WO9604545

(56) Entgegenhaltungen:
- EP-A- 0 387 793
- DE-A- 4 341 063
- US-A- 4 515 165

## Beschreibung

Gegenstand der Erfindung ist ein Verfahren zur optischen Charakterisierung von innerer Struktur oder/und Zusammensetzung einer ausgedehnten streuenden Probe mit einer Anordnung von einer oder mehreren Lichtquellen und einem oder mehreren Lichtdetektoren sowie ein hierfür geeigneter Apparat.

In der medizinischen Diagnostik wird schon seit geraumer Zeit versucht, innere Strukturen von Körpern zur Erkennung von Krankheiten zu verwenden. Begonnen hatte diese Entwicklung durch Beleuchtung eines Objektes mit ausgewählten Wellenlängen oder mit weißem Licht und Aufzeichnung der reflektierten Intensität (non-invasive methods for the quantification of skin functions; P.J. Frosch und A.M. Klingman (Herausgeber), Springer Verlag Berlin/Heidelberg, 1993, Seiten 3 bis 24 und 25 bis 41). Ein Nachteil dieser Methode ist, daß nur Oberflächeneigenschaften detektiert werden, da die Information durch Absorptions- oder Reflektionseigenschaften der Oberfläche bestimmt wird. Ein weiterer Nachteil dieser Methode ist die schlechte Reproduzierbarkeit bei quantitativer Auswertung.

Auch die beim Einscannen von beschriebenem Papier verwendeten Scanner tasten nur die Oberfläche ab, da das gemessene, aufgrund der kontinuierlichen Beleuchtung und der von Lichtquelle und Empfänger im wesentlichen an der Oberfläche reflektierte Licht nicht in das Papier eindringen soll und kann.

Sonographische Verfahren beruhen auf der Unterscheidbarkeit unterschiedlicher Strukturen, wie z. B. Gewebetypen aufgrund ihres unterschiedlichen Reflektionsfaktors für Schallwellen, der proportional der akustischen Impedanz ist (I. Krestel (Herausgeber), Bildgebende Systeme für die medizinische Diagnostik; Siemens AG, Berlin, München, 2. Auflage 1988, Seite 183 ff.). Mittels Sonographie können zwar Informationen aus dem Inneren der Probe gewonnen werden, diese sind jedoch ausschließlich von der mechanischen Beschaffenheit (Dichte) abhängig. Dies bedeutet, daß lediglich Strukturen mit stark verschiedenen akustischen Impedanzen unterschieden und dargestellt werden können. Für die verschiedenen weichen Gewebeteile sind aber die Unterschiede der akustischen Impedanz bzw. des Reflektionsfaktors sehr gering und zeigen keine oder nur sehr schwache Kontraste.

Die Verwendung von Licht mit Wellenlängen im Bereich des nahen Infrarot (NIR) besonders in der in-vivo-Analytik ermöglicht aufgrund der optischen Gewebeeigenschaften (Streu- und Absorptionskoeffizient) die Durchdringung dickerer Gewebebereiche (einige Millimeter bis mehrere Zentimeter) zerstörungsfrei (im Gegensatz zu Röntgenstrahlen). Bei den bisher bekannten Verfahren ist es jedoch für eine Bildgebung aus den gemessenen Lichtintensitäten notwendig, durch den Einsatz von zeitlich hoch aufgelösten Meßverfahren (im ps-Bereich) und/oder durch erheblichen Rechenaufwand den Weg der Photonen basierend auf verschiedenen Modellannahmen mathematisch zurückzuverfolgen. Die Meßapparatur besteht bei diesen Verfahren im allgemeinen aus einem ultrakurzgepulsten Laser hoher Strahlungsleistung, einem komplexen optischen Aufbau zum lateralen Abtasten der Probenoberfläche und einem ultraschnellen Detektor. Als Laser werden z. B. Moden-gekoppelte Gasionenlaser (Ar, Kr), welche synchron Farbstofflaser pumpen, eingesetzt. Zur Detektion eignen sich besonders Streak Kameras, Microchannel Photomultiplier oder Kerr-Shutter (L. Wang et al.; Ballistik Imaging ofBiomedical Samples using ps optical Kerrgate; SPIE Vol. 1431, Time Resolved Spectroscopy and Imaging ofTissues (1991), Seite 97 ff.).

Sowohl die Handhabung als auch die Kosten schränken den Einsatz dieser Apparate auf hochqualizierte und hochspezialisierte optische Labors ein. Äquivalente Halbleiterbauelemente, die für den gleichen Meßzweck eingesetzt werden können, und welche die erforderliche Miniaturisierung erlauben, sind derzeit nicht verfügbar bzw. die zukünftige Verfügbarkeit ist noch nicht absehbar. Zu den apparativen Schwierigkeiten kommt noch das Problem bei der Bildrekonstruktion hinzu. Da die Photonen auf unzähligen Wegen von der Lichtquelle zum Detektor wandern, ist ein eindeutiger Rekonstruktionsalgorithmus, wie z. B. bei der Computertomographie, nicht formulierbar. Obwohl erste Ansätze vorhanden sind (S.R Arridge et al.; New results for the development of infra-red absorption imaging; Proceedings of SPIE - The International Society of Optical Engeneering, Vol. 1245, Seite 92 - 103) hat noch kein Verfahren erfolgreich eine Rekonstruktion zeigen können. Außerdem sind diese Verfahren aufgrund der derzeit notwendigen Rechenzeiten für den Einsatz in der klinischen Diagnostik nicht geeignet.

In der EP-A 0 387 793 ist ein Sensor beschrieben, welcher auf ein Hautareal aufgelegt werden kann, und mit dessen Hilfe ein optisches Bild des darunter liegenden Gewebebereiches erzeugt werden kann. Dieser Apparat ist jedoch für die Untersuchung größerer Körperflächen nicht geeignet, da hierfür eine sehr große Zahl von Detektoren verwendet werden müßte. Darüber hinaus ist die optische Auflösung des erzeugten Bildes nicht befriedigend.

In der DE-A-4341063 ist ein Verfahren zur Bestimmung von Dichteverteilungen beschrieben, bei dem durch das Gewebe hindurchgetretenes Licht hinsichtlich Phase und Amplitude hochfrequent modulierter Strahlung ausgewertet wird. Es wird eine U-förmige Meßanordnung offenbart, bei der sich Strahlungsquelle und Detektoreinheit auf gegenüberliegenden Seiten der Probe befinden. Durch die U-förmige Anordnung ist es möglich, eine Dickemessung der untersuchten P-obe vorzunehmen, um die Dicke in die Auswertung mit einzubeziehen. Weiterhin ist es beschrieben, daß in der U-förmigen Meßanordnung eine Scan-Einheit integriert ist, mit der eine ortsaufgelöste Registrierung transmittierter Strahlungsanteile möglich ist.

Aufgabe der vorliegenden Erfindung war es daher, einen Apparat bzw. ein Verfahren zur Verfügung zu stellen, mit dem auch die innere Struktur von streuenden Proben in hoher örtlicher Auflösung erfaßt werden kann.

Gegenstand der Erfindung ist daher ein Apparat oder eine Vorrichtung zur optischen Charakterisierung von innerer Struktur oder/und Zusammensetzung einer ausgedehnten streuenden Probe gemäß Anspruch 1. Ebenfalls Gegenstand der Erfindung ist ein Verfahren zur optischen Charakterisierung dieser Strukturen gemäß Anspruch 7.

Unter einem Apparat zur optischen Charakterisierung wird ein Gerät verstanden, welches eine Charakterisierung mit Hilfe der Einstrahlung von Licht aus einer Lichtquelle und Detektion des von der Probe auf einen Lichtdetektor fallenden Lichtes durchführt. Der Apparat kann erfindungsgemäß sowohl eine als auch mehrere Lichtquellen enthalten. Außerdem ist es bevorzugt, daß der Apparat mehrere Lichtdetektoren enthält.

Optische Charakterisierung schließt dabei alle Meßverfahren ein, die eine bestimmte Eigenschaft des Lichts detektieren, die von der inneren Struktur der Probe beeinflußt wird. Dies kann z. B. eine Messung der Intensität (Schwächung der Lichtintensität durch Absorption und Streuung, z. B. analog PCT/DE 93/01058), eine Messung des Polarisationsgrades des Lichtes (Depolarisation von polarisiert eingestrahltem Licht durch z. B. Streuprozesse) oder die Laufzeit der Photonen (z.B. analog DE-A-43 37 570) sein.

Unter einer ausgedehnten Probe wird hier eine Probe verstanden, die eine 2-dimensionale Oberfläche aufweist und deren innere Struktur 3-dimensional inhomogen ist. Bevorzugt sind solche Proben mindestens 1 cm lang und mindestens 1 cm breit, besonders bevorzugt liegt die Ausdehnung zwischen 2 und 15 cm in der Länge und 2 bis 5 cm in der Breite.

Damit das eingekoppelte Licht ohne direktes Übersprechen von Lichtquelle durch die Probe zum Detektor gelangen kann, muß die Probe außerdem das eingedrungene Licht streuen. In diesem Fall kann das durch eine die Probe begrenzende Grenzfläche eindringende Primärlicht in die Probe eindringen, in der Probe entlang einem Lichtweg propagieren und wiederum aus dieser Probe durch eine von der ersten Grenzfläche entfernten Grenzfläche als Sekundärlicht austreten. Auf dem Lichtweg finden die in PCT/DE 93/01058 beschriebenen Vielfachstreuungen statt.

Die Streuung kann sowohl an in der Probe enthaltenen Partikeln als auch anderen inneren Strukturen, wie Zellwänden, geschehen. Diese Eigenschaften einer Probe erfüllen im besonderen Maß menschliche und tierische Gewebe, insbesondere die Haut und die darunter liegenden Gewebebereiche, bevorzugt bis zu einer Tiefe von ca. 25 mm.

Die Lichtquelle wird so gewählt, daß sie Licht ausstrahlt, welches je nach gewähltem Meßverfahren eine bekannte Intensität oder einen bekannten Polarisationsgrad oder eine bekannte Intensitätsmodulation bekannter Phasenmodulation aufweist und welches die oben genannte Eindringbedingung erfüllt und von der Probe gestreut wird. Zur Ermittlung der optimalen Wellenlänge geht der Fachmann folgendermaßen vor:

Zur bevorzugten Erfassung von absorbierenden Strukturen wie z. B. Blutgefäße, werden die Wellenlängen ausgewählt, bei denen der Extinktionskoeffizient der interessierenden Struktur verglichen mit der Umgebung groß ist, d. h. daß dadurch ein hoher Kontrast entsteht. Beispielsweise hat sich bei der Detektion von Blut eine Wellenlänge von ca. 650 nm bewährt.

Zur bevorzugten Erfassung von streuenden Strukturen wird eine Wellenlänge ausgewählt, bei der keine oder möglichst geringe spezifische Absorptionsbanden liegen, die das Ergebnis verfälschen können, da beispielsweise die Abnahme der Intensität durch stark streuende Strukturen im Lichtweg oder auch durch Absorption verursacht sein kann.

Zur Untersuchung von Objekten sind Wellenlängen im ultravioletten sichbaren und infraroten Wellenlängenbereich zwischen 200 und 10000 nm geeignet.

Für die menschliche Haut haben sich Wellenlängen aus dem Bereich 400 bis 2500 nm als geeignet erwiesen. Besonders bevorzugte Wellenlängen liegen in den Bereichen 400 bis 1300 nm. Lichtquellen, mit denen Licht mit dieser Wellenlänge erzeugt werden kann, sind dem Fachmann bekannt. Besonders geeignet sind Laserdioden oder LED, da sie so klein sind, daß eine Vielzahl von Lichtquellen auf einer relativ kleinen Fläche des Apparates angeordnet werden können.

Als Lichtdetektoren haben sich insbesondere Photodioden oder CCDs als besonders geeignet erwiesen, da auch sie eine enge Bauweise zulassen.

Unter einem Wegaufnehmer wird ein Bauteil verstanden, welches bei Bewegung des Apparates auf der Oberfläche der Probe den zurückgelegten Weg festhält und an eine Steuereinheit weitergibt. Solche Wegaufnehmer sind dem Fachmann prinzipiell bekannt.

Geeignet sind beispielsweise mechanische Geräte, die mittels einer Winkelcodierung arbeiten. Für eine geradlinige Wegaufnahme sind beispielsweise Walzenwegaufnehmer bevorzugt. Soll der Weg nichtlinear aufgenommen werden, eignen sich Kugelwegaufnehmer, wie sie beispielsweise in sogenannten Mäusen zur Steuerung von PCs Einsatz finden. Diese Wegaufnehmer werden dazu benutzt, jedem an einem Lichtdetektor gemessenen Meßwert einen Meßort zuzuordnen.

Bevorzugt weist eine erfindungsgemäße Anordnung für eine geradlinige Erfassung mindestens zwei Walzen auf, die in Bewegungsrichtung vor und nach den Lichtquellen und Lichtdetektoren liegen. Diese Walzen weisen an ihrer Oberfläche ein Material auf, welches ein Abrutschen der Walze auf der Probenoberfläche erschwert. Bevorzugt haben die Walzen eine Oberfläche aus Gummi. Es hat sich ferner bei elastischen Proben, wie z. B. Gewebe, als bevorzugt erwiesen, wenn die Oberfläche der Walzen 0.1 bis 5 mm, besonders bevorzugt 0.5 bis 2 mm über die der Probe zugewandte Oberfläche des Apparates hinausragt, welche die Lichtquellen und Lichtdetektoren enthält. Dieser Abstand stellt sicher, daß bei üblichen Andruckkräften des Apparates auf die menschliche Haut möglichst kein Zwischenraum zwischen dieser Oberfläche des Apparates und der Oberfläche der Probe vorhanden ist. Diese Anordnung vermeidet weitestgehend Störungen, die durch die Reflektion des eingestrahlten Lichts an der Oberfläche der Haut bzw. direkt übertretene Strahlung von einer Lichtquelle auf einen Detektor sich ergeben können.

Die Zuordnung der Meßwerte (Intensitäten zu Meßort) wird in einem elektronischen Auswerteteil vorgenommen. Die Werte können in einem Speicherteil gespeichert und bei Bedarf abgerufen werden. Aus der Gesamtheit der Meßwerte fiir die auf dem zurückgelegten Weg liegenden Meßorte läßt sich pro Lichtquelle-/Lichtdetektor-Paar ein Intensitätsprofil angeben. Für den Fall, daß mehrere Lichtquelle-/Lichtdetektor-Paare ausgewertet werden, erzeugen Lichtdetektoren, die nicht auf der gleichen Wegstrecke liegen, ebenfalls weitere Intensitätsprofile, die die innere Struktur der Probe zwischen Lichtquelle und Lichtdetektor beschreiben. Durch Verwendung einer Vielzahl von Lichtdetektoren ergibt sich daher ein 3-dimensionales Bild, wenn die Intensität in Abhängigkeit vom zurückgelegten Weg und vom Detektorort aufgetragen wird.

Neben mechanischen Wegaufnehmern sind auch optische Wegaufnahmeverfahren oder auch mathematische Verfahren zur Wegaufnahme geeignet.

So kann z. B. durch Aufbringen eines regelmäßigen Hell-Dunkel Rasters mit bekannter Dimension auf die Probe durch die Messung der Hell und Dunkelzonen vergleichbar mit einem Barcodeleser der Ort eindeutig bestimmt werden. Dazu können entweder gesondert Detektoren angebracht werden, die außerhalb des eigentlichen Meßfeldes liegen oder das Raster kann direkt mit der Messung aufgezeichnet werden, wenn das Raster auf der Probe direkt oder durch einen bei den verwendeten Wellenlängen transparenten Träger auf die Probe aufgebracht wird.

Ein mathematisches Verfahren der Wegaufnahme ist bei der Verwendung von mehreren gleichartigen Meßpaaren möglich, die so angeordnet sind, daß diese beim Verfahren des Apparates in Bewegungsrichtung die gleiche Stelle überstreichen und dadurch das gleiche Intensitätsprofil messen, jedoch zeitversetzt in Abhängigkeit von der Bewegungsgeschwindigkeit. Bei gegebenen Abstand zwischen diesen beiden Meßpaaren kann durch die zeitliche Korrelation dieser beiden Meßkurven die Geschwindigkeit und damit bei bekannter Zeit der Ort errechnet werden.

Im erfindungsgemäßen Apparat ist die Geometrie der Anordnung der Lichtquellen, Lichtdetektoren und des Wegaufnehmers während der Messung bevorzugt konstant. Die Anordnung kann prinzipiell aus einer einzigen Lichtquelle und einem dazugehörigen Lichtdetektor bestehen, so daß wie oben ausgeführt, ein Intensitätsprofil I als Funktion des Ortes erzeugt wird. In einer weiteren Möglichkeit wird zwar nur eine Lichtquelle verwendet, jedoch viele Detektoren, die sich entweder durch einen unterschiedlichen Abstand zur Lichtquelle und/oder durch einen unterschiedliche Winkel zwischen Lichtquelle-Detektor und Bewegungsrichtung auszeichnen.

Im bevorzugten Fall enthält die erfindungsgemäße Anordnung jedoch mehrere Lichtquellen und mehrere Lichtdetektoren. Für die Durchführung einer Messung wird bevorzugt zu einer Zeit immer nur eine Lichtquelle eingeschaltet und die auf einen Detektor oder mehrere Detektoren einfallende Lichtmenge detektiert. Wenn mehrere Lichtquellen gleichzeitig eingeschaltet werden, muß sichergestellt sein, daß die von einem Detektor gemessene Lichtmenge im wesentlichen von einer Lichtquelle ausgeht, bevorzugt zu 90 %, besonders bevorzugt mehr als 99 %. Dies gilt sinngemäß auch fiir den Einfluß von Fremdlicht, z. B. durch Tageslicht oder Beleuchtungseinrichtungen. Daher sollten die Einschaltzeiten der Lichtquellen zeitlich getaktet werden. Die Kenntnis dieser Einschalttakte erlaubt eine genaue Zuordnung von eingeschalteter Lichtquelle und den Detektoren, die zu dieser Zeit ausgelesen werden. Die Dauer dieser Takte ist bevorzugt für alle Lichtquellen gleich groß und während einer Messung konstant. Die Einschaltzeiten - oder reziprok die Taktfrequenzenhängen ab von der gewünschten maximalen Bewegungsgeschwindigkeit, der Ortsauflösuing in Bewegungsrichtung, der Anzahl der Lichtquellen und Detektoren, die bei gegebener Bewegungsgeschwindigkeit innerhalb der gegebenen Ortsauflösung in Bewegungsrichtung eingeschaltet und ausgelesen werden müssen. Als Bewegungsgeschwindigkeiten im Handbetrieb haben sich Geschwindigkeiten zwischen 1 und 20 cm/s als zweckmäßig gezeigt. Beim Einsatz des erfindungsgemäßen Apparates mit mechanischen Hilfen zur Bewegungssteuerung (z. B. Motoren) können je nach gewünschter Ortsauflösung auch wesentlich höhere oder niedrigere Geschwindigkeiten erzielt werden und sinnvoll sein. In dem Geschwindigkeitsbereich im Handbetrieb sind Taktfrequenzen für z. B. 16 Lichtquellen und 16 Detektoren zwischen 10 Mhz und 1 kHz, besonders bevorzugt zwischen 1 MHz und 20 kHz besonders sinnvoll. Damit ergeben sich Auflösungen in Bewegungsrichtung unter 0.1 mm, d. h., unterhalb der Größe der interessierenden Strukturen.

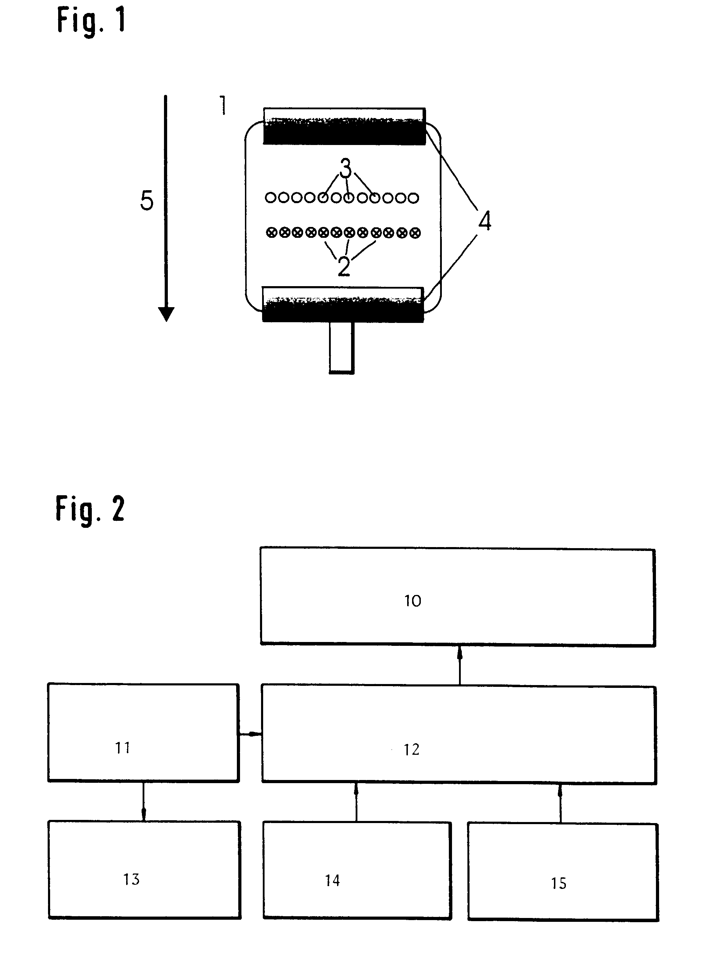

In einer vorteilhaften Ausführungsform sind daher die Lichtquellen und Lichtdetektoren jeweils in Paaren, die aus jeweils einer Lichtquelle und einem Lichtdetektor bestehen angeordnet, wobei die Lichtquellen- und Lichtdetektoren der Paare jeweils auf einer gedachten Linie im wesentlichen senkrecht zur Bewegungsrichtung der Anordnung liegen (s. Fig. 1).

Gemäß Figur 1 werden bevorzugt die Lichtquellen zeitlich versetzt aktiviert und die auf einen bestimmten Detektor einfallende Lichtmenge detektiert. Der einer aktivierten Lichtquelle zugeordnete Detektor bildet mit dieser ein Paar.

Bevorzugt ist der Abstand zwischen Lichtquelle und Lichtdetektor jeden Paares und der Winkel zwischen zwischen Lichtquelle-Detektor und Bewegungsrichtung gleich groß. Für die bevorzugten Proben und Lichtcharakteristiken hat sich ein Abstand d von 1 bis 50 mm, besonders bevorzugt von 2.5 bis 20 mm als vorteilhaft erwiesen.

Lichtquelle und Lichtdetektor jeden Paares befinden sich an der gleichen Grenzfläche der Probe, d. h. es wird in Reflexion gemessen. Dabei darf der Begriff Reflexion im Hinblick auf den diffusen Charakter des Sekundärlichtes natürlich nicht so verstanden werden, daß das Sekundärlicht mit einer stark dominierenden Vorzugsrichtung aus der Probe austritt.

In den Figuren werden die Längen und Abstände X und Y in Millimetern angegeben. Die Intensitäten I werden in relativen Einheiten angegeben.

Die in Figur 1 gezeigte Anordnung 1 weist neben den Lichtquellen 2 und Lichtdetektoren 3 zwei Walzen 4 auf, von denen mindestens eine als Wegaufnehmer dient. Die geeignete Anordnung ist daher für die Aufnahme mehrerer linearer Intensitätsprofile entsprechend den zurückgelegten Wegen der Detektoren in Bewegungsrichtung 5 geeignet.

Neben dieser Anordnung enthält der erfindungsgemäße Apparat weitere für den Verwendungszweck hilfreiche Bauteile. Es ist hierbei zu berücksichtigen, daß diese Bauteile sowohl fest, flexibel oder auch über Kommunikationswege miteinander verbunden sein können. Der resultierende ein- oder mehrteilige Apparat sollte für eine ordnungsgemäße Funktion neben der beschriebenen Anordnung von Lichtquellen, Lichtdetektoren und Wegaufnehmer eine Ansteuerungselektronik für Lichtquellen und Detektoren, einen Verstärker, ein Display, Schreiber oder Analog-Digitalwandler und Computer für die Weiterverarbeitung der erhaltenen Daten enthalten. Bevorzugt sind alle oben genannten Bauteile mit Ausnahme der Anordnung 1 in einem Standgerät untergebracht, während die Anordnung 1 in einem relativ kleinen Handgerät untergebracht ist. Die Verbindung zwischen diesen beiden Elementen kann entweder mechanisch flexibel über eine Kabelverbindung oder über elektromagnetische Strahlung hergestellt sein.

Figur 2 zeigt ein Blockschaltbild eines erfindungsgemäßen Aufbaus.

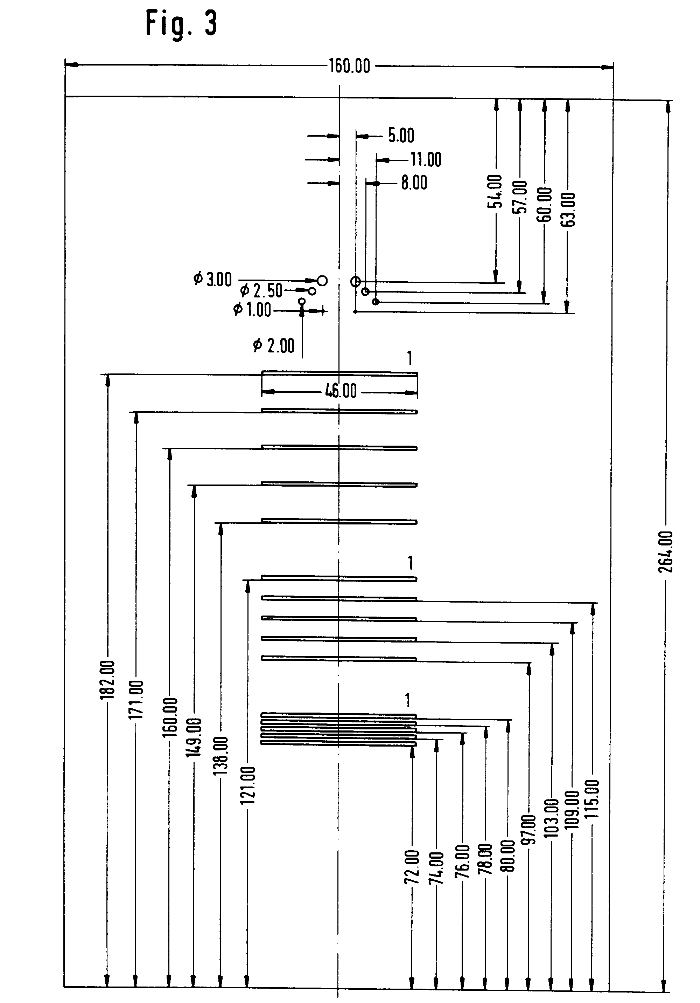

Figur 3 beschreibt einen Testobjekt aus streuendem Material (Dicke ca. 20 mm aus Teflon), in dem mechanische Inhomogenitäten in Form von definierten Vertiefungen (Tiefe 4.5 mm (1) bzw. 2.5 mm (Rest)) in unterschiedlichen Abständen vorhanden sind.

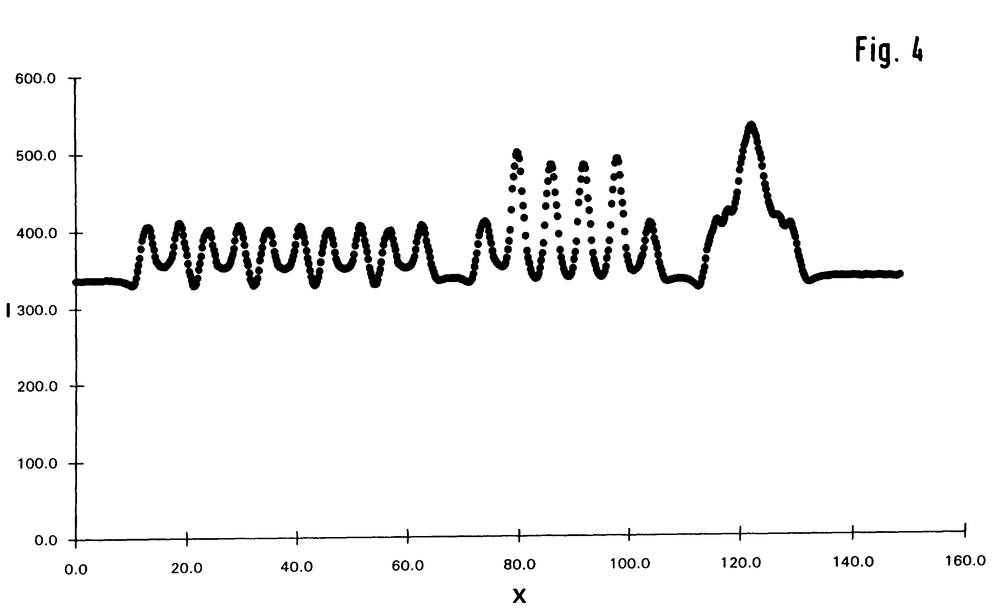

Figur 4 zeigt ein 1-dimensionales Intensitätsprofil I als Funktion des Ortes, das mit einem erfindungsgemäßen Apparat, bei dem die Abstände zwischen Lichtquelle und Detektor ca. 7 mm betragen, durch Vermessung des in Fig. 3 beschriebenen Testobjekt erzeugt wurde.

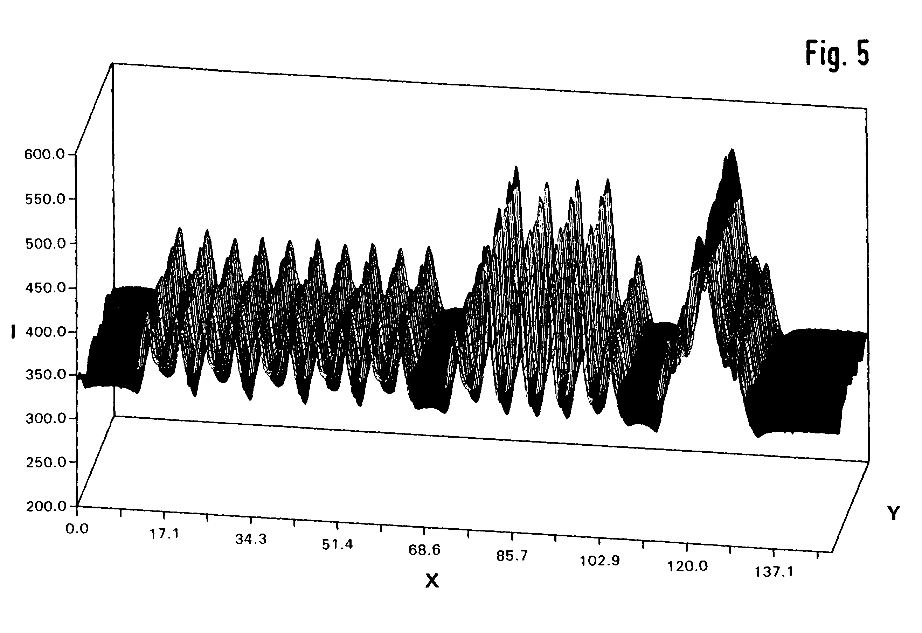

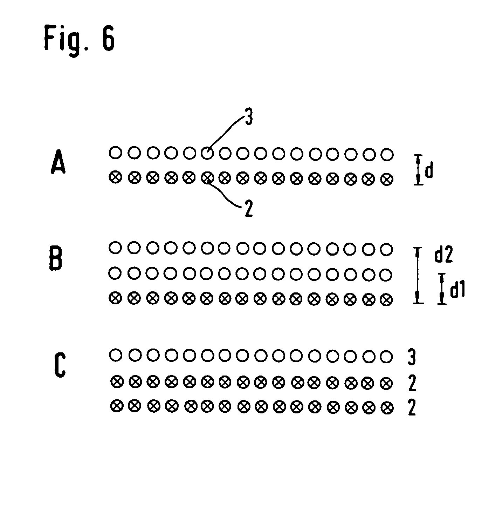

Figur 5 zeigt ein 2-dimensionales Intensitätsprofil, das mit dem gleichen Testobjekt erzeugt wurde. Der erfindungsgemäße Apparat bestand dabei aus einer Reihe von 14 Lichtquellen und 14 Detektoren entsprechend der Anordnung A in Fig 6 mit einem Abstand d von 7 mm.

In Figur 6 sind drei Möglichkeiten zur Anordnung von Lichtquellen/Lichtdetektoren gezeigt. In Anordnung A sind sowohl die Lichtquellen (2) als auch die Lichtdetektoren in jeweils einer gedachten Linie angeordnet. Der Abstand zwischen den beiden Linien ist mit d bezeichnet. In Anordnung B ist die Auswertung für zwei unterschiedliche Abstände d1 und d2 dadurch möglich, daß eine zweite Reihe von Lichtdetektoren vorgesehen ist. Derselbe Effekt läßt sich durch Verwendung zweier Reihen von Lichtquellen mit nur einer Reihe von Lichtdetektoren erreichen (Anordnung C).

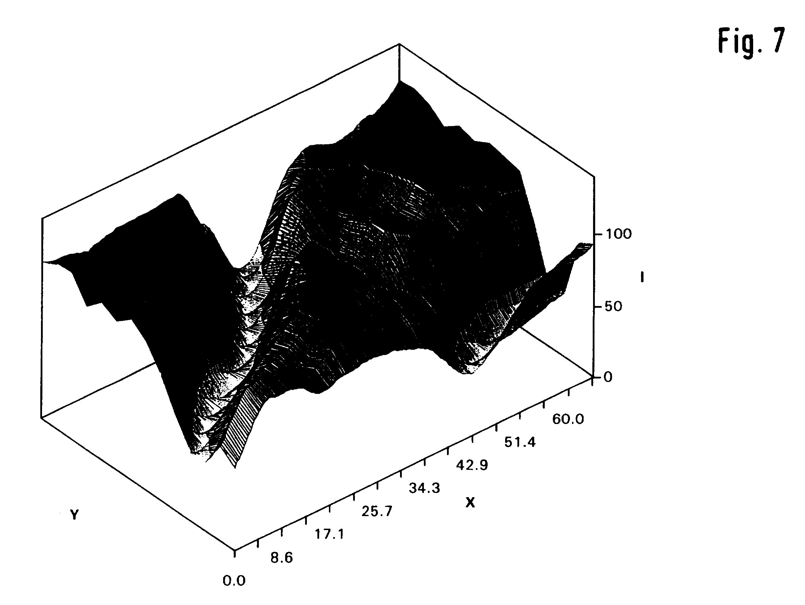

In Figur 7 ist eine 2-dimensionale Darstellung der Intensität als Funktion der Verfahrstrecke und der Lichtquelle-/Detektorposition, die mit einem Apparat gemäß Figur 1 an menschlicher Haut gewonnen wurde, gezeigt. Aufgetragen sind die Intensitätsprofile von 14 Detektoren über eine Wegstrecke von 60 mm. Der Verlauf einer Vene (niedrige Intensitäten, dunkle Stellen) ist deutlich zu erkennen.

Da die Lichteigenschaft nach Durchdringen der Probe in Reflektion gemessen wird, wird das 2-dimensionale Intensitätsprofil von Parametern bestimmt, die die optischen Eigenschaften des untersuchten Objektes beeinflussen.

Die gemessenen Intensitätsprofile, die zunächst nur eine relative Zuordnung erlauben, können dadurch zur absoluten Feststellung der inneren Struktur eines vorgegebenen Bereiches verwendet werden, daß der Ort des Beginns oder Endes der Messung auf der Probe festgehalten wird. Damit kann die relative Intensitätsmessung bestimmten Orten der Probe zugeordnet werden. Zweckmäßigerweise definiert man auf dem Apparat einen Koordinaten-Nullpunkt für die exakte Wiederfindung in den verschiedenen Bewegungsrichtungen. Vorteilhaft ist dies die Position der Lichtquellen oder der Detektoren.

Weiterer Gegenstand der Erfindung ist ein Verfahren zur optischen Charakterisierung von innerer Struktur und/oder Zusammensetzung einer ausgedehnten streuenden Probe, gemäß Anspruch 7, bei dem ein Apparat, der eine oder mehrere Lichtquellen und einen oder mehrere Lichtdetektoren enthält, über die ausgedehnte Probe hinwegbewegt wird. Die Bewegung der Anordnung aus Lichtquellen und Lichtdetektoren wird über einen Wegaufnehmer festgehalten. Während der Bewegung des Apparates wird das auf die Lichtdetektoren einfallende Licht detektiert und das erhaltene Signal festgehalten. Um die gemessenen Signale bestimmten Orten der Probe zuordnen zu können, werden die gemessenen Intensitäten mit der zurückgelegten Wegstrecke korreliert. Dies geschieht zweckmäßigerweise in einem Computer. Die resultierenden Intensitätsprofile können numerisch oder graphisch dargestellt werden.

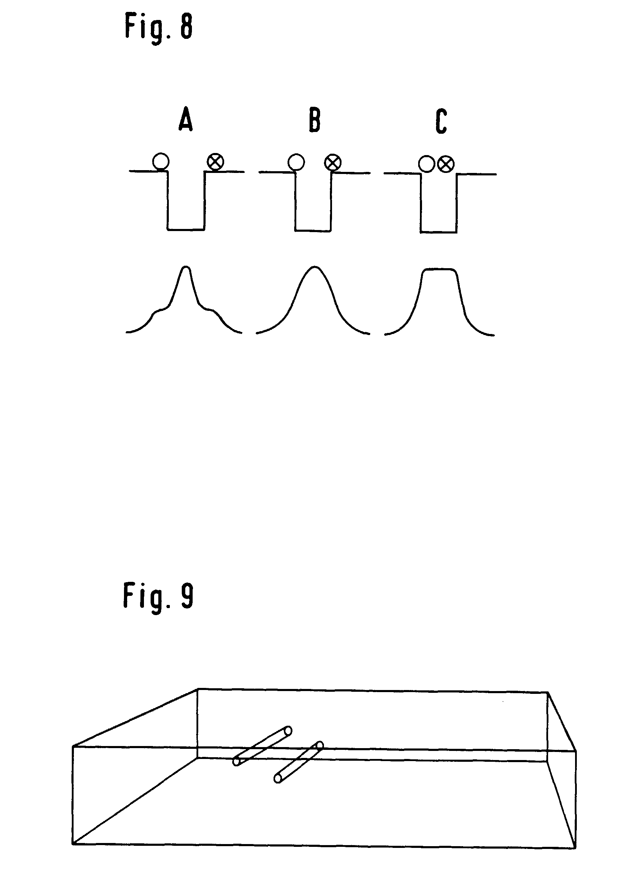

Auf die mögliche Auflösungsgenauigkeit hat außerdem der Abstand d zwischen Lichtquelle und Lichtdetektor einen Einfluß. In Figur 8 sind beispielhafte Intensitätsverläufe für die Fälle gezeigt, daß der Abstand d größer ist als eine innere Struktur, d ungefähr gleich ist wie die innere Struktur und d sehr viel kleiner ist als die innere Struktur. Als innere Struktur wird in diesem Fall eine Spalte in der Oberfläche der Probe gezeigt, über die die Anordnung hinwegbewegt wird. Durch die Verwendung von unterschiedlichen Wellenlängen und/oder Lichtquelle- Detektorabständen können bestimmte Eindringtiefen bevorzugt werden und die Spezifität für bestimmte Komponenten erhöht werden. Zur Untersuchung von Gefäßen eignen sich z. B. Wellenlängen, bei denen Hämoglobin eine Absorption zeigt. Gefäßreiche Stellen zeigen dann eine höhere Absorption, die zu einer geringeren gemessenen Lichtintensität bei den Lichtquelle-/Detektor-Paaren, die sich über dem Gefäß befinden, führt.

In den Figuren 9 - 13 ist das erfindungsgemäße Verfahren zur Anwendung an einem weiteren, detaillierteren Modellsystem gezeigt. Ein streuender Kunststoffblock wurde mit absorbierenden Strukturen in unterschiedlicher Tiefe versehen und mit einem absorbierenden Medium zur Simulation von absorbierenden Blutgefäßen in Gewebe gefüllt. Die Bohrungen haben einen Durchmesser von 1 mm und liegen parallel zur Oberfläche in einem Abstand von 2,8 und 5,0 mm zur Oberfläche und sind etwa 20 mm lang (Fig. 9).

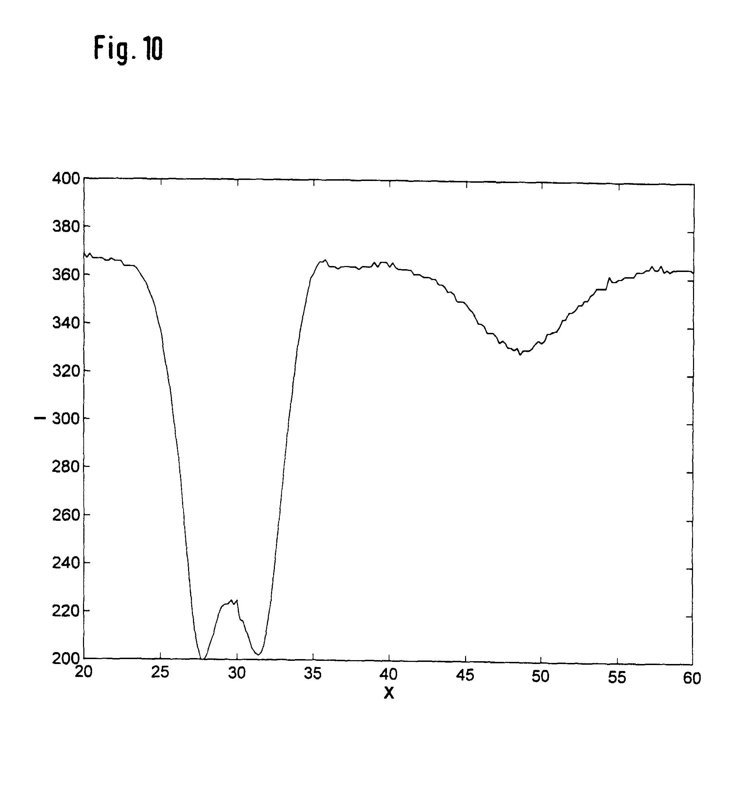

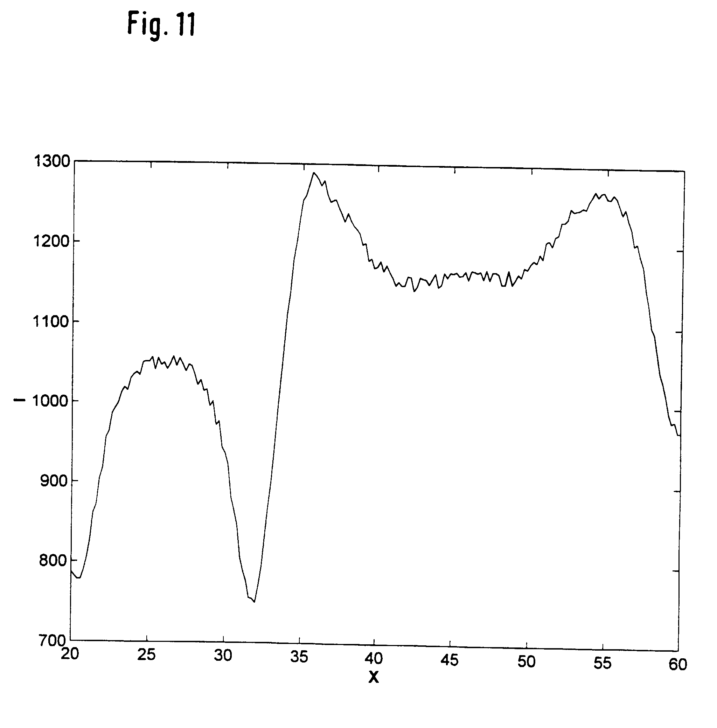

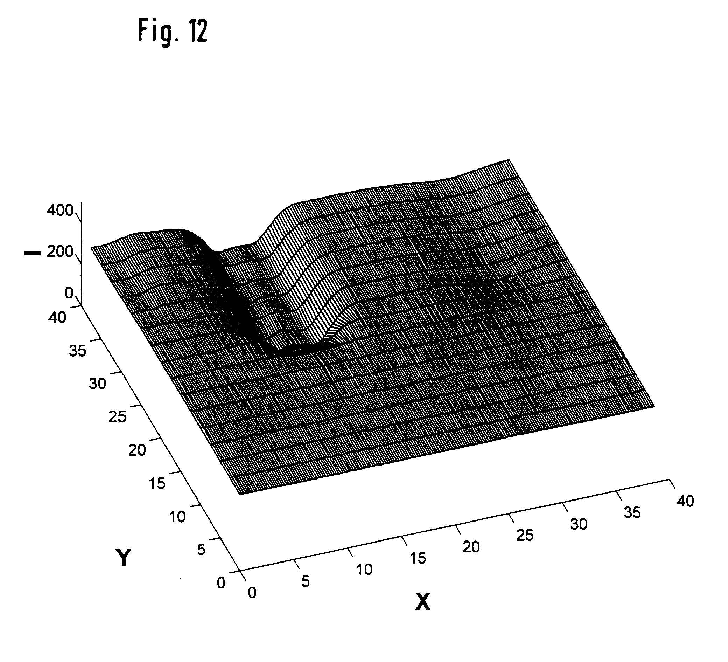

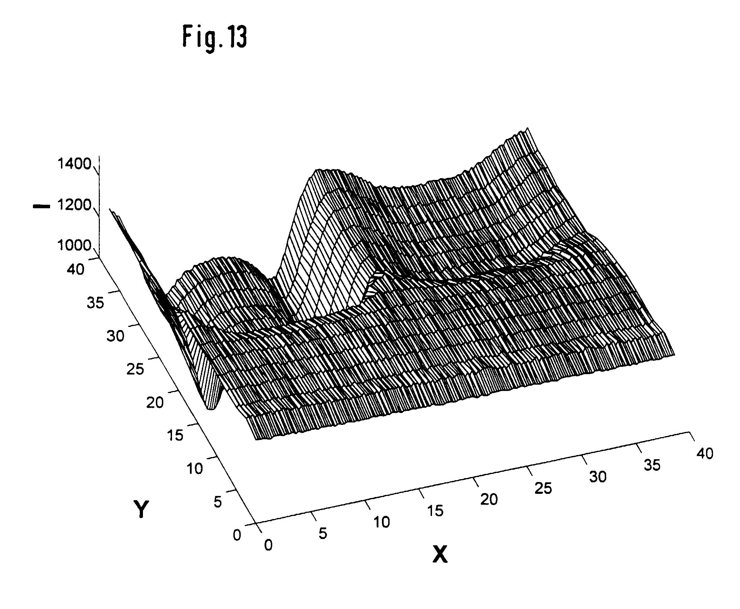

Durch die Form der Intensitäts-Wegverläufe können Aussagen über Lage, Tiefe und Gestalt der Strukturen getroffen werden. Gezeigt ist in Figur 10 der Intensitäts-Wegverlauf für ein ausgewähltes Lichtquellen-Detektor-Paar mit dem Abstand 7 mm. Figur 11 zeigt den Signalverlauf für die gleiche Lichtquelle jedoch für einen Detektor im Abstand von 14 mm. Den Signalen bei 25 mm bzw. 50 mm Weg entsprechen die Bohrungen mit 2,8 bzw. 5,0 mm Tiefe. Aus der unterschiedlichen Form der Signale kann auf die Ausdehnung und die Tiefe der absorbierenden Bohrungen geschlossen werden. Dabei zeigen beispielsweise nahe an der Oberfläche liegende Strukturen bei kleinem Lichtquellen-Detektor-Abstand einen steilflankigen Signalabfall, die tieferliegende Struktur wird als relativ schwacher und breiter Signalabfall abgebildet. In Figur 12 und 13 ist der Intensitätsverlauf über die gesamte Scannbreite für 7 mm und 14 mm Abstand zwischen Lichtquelle und Detektor dargestellt.

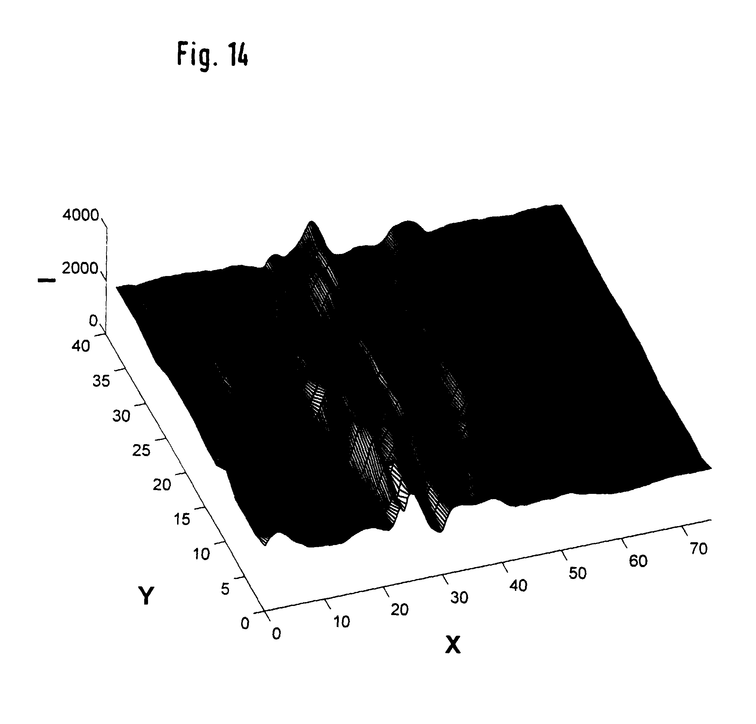

In Figur 14 ist gezeigt, daß das erfindungsgemäße Verfahren auch zur in-vivo-Messung geeignet ist. Figur 14 zeigt eine Messung von Schädigungen des Bindegewebes durch Überdehnung (sog. Schwangerschaftsstreifen). Aufgetragen ist die reflektierte Intensität bei einem Lichtquellen-Detektor-Abstand von 7 mm als Funktion des Weges. Zwischen 0 und 50 mm Weg sowie ab 100 mm Weg sind senkrecht zur Scannrichtung verlaufende, streifenförmige Veränderungen der Haut zu erkennen. Zwischen 50 und 100 mm Weg handelt es sich um normales Gewebe. Der Einsatz erfindungsgemäßer Geräte erlaubt sowohl die Dokumentation solcher Befunde als auch deren Kontrolle im Verlauf einer Therapie. Im Gegensatz zu einer rein visuellen Beurteilung kann aus den gezeigten Intensitätsprofilen der Verlauf und die Ausdehnung der Gewebeveränderungen im Innern des Hautgewebes nichtinvasiv beurteilt werden.

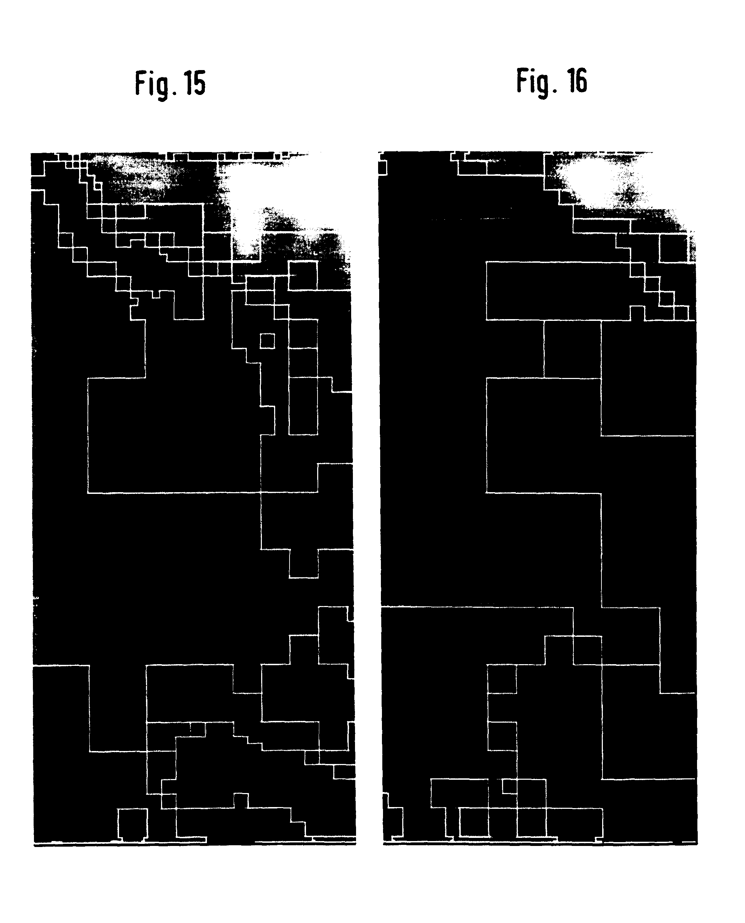

In den Figuren 15 ist gezeigt, daß das erfindungsgemäße Verfahren zur Bewertung der Homogenität von Gewebe eingesetzt werden kann. Zur Bestimmung der optischen Eigenschaften der Haut ist eine Bewertung bezüglich der Homogenität der untersuchten Stellen wichtig, da aufgrund des streuenden Charakters der Haut nicht ein definierter Ort, sondern ein unscharf begrenztes Volumen vermessen wird. Die abgeleiteten optischen Größen stellen also Mittelwerte über dieses Volumen dar. Daher ist es notwendig, die Homogenität des untersuchten Gewebsbereiches zu kennen, um Verfälschungen des Mittelwertes durch Inhomogenitäten auszuschließen.

In Figur 15 und 16 ist die gemessene 2-dimensionale Intensitätsverteilung einer Hautstelle in einer Grauwertdarstellung gezeigt. In Figur 15 betrug der Lichtquellen-Detektor-Abstand 7 mm, in Figur 16 14 mm. Durch die Weiterverarbeitung der Daten kann die untersuchte Hautstelle in Bereiche ähnlicher Intensitäten aufgeteilt werden. Dabei kann nach einem Zonenwachstumsverfahren vorgegangen werden. Dazu vergleicht man im ersten Schritt einen beliebigen Intensitätswert der 2-dimensionalen Datenstruktur I(x,y) mit den Nachbarwerten I(x+1, y+1), I(x, y+1), I(x-1, y+1) usw. Diejenigen Nachbarwerte, die ein bestimmtes Vergleichskriterium erfülle,z. B. innerhalb eines bestimmten Wertebereiches mit dem ersten Punkt liegen, werden zu einer Fläche zusammengefaßt. Daraus resultiert eine erste Fläche mit ähnlichen Intensitätswerten. Zur Bestimmung weiterer Flächen muß ein neuer Startpunkt außerhalb der bereits ermittelten Fläche oder Zone ermittelt werden und danach erfolgt wieder die oben beschriebene Vergleichsprozedur. Eine Messung der optischen Eigenschaften innerhalb einer solchen ausgezeichneten Fläche wäre vorteilhaft gegenüber einer Messung auf der Grenze der gezeigten Flächen. Außerdem zeigt sich durch einen Vergleich zwischen Figur 15 und 16, daß das untersuchte Gewebe bei unterschiedlichen Lichtquellen-Detektorabständen unterschiedliche homogene Bereiche hat.

Ebenfalls Gegenstand der Erfindung sind vorteilhafte Verwendungsmöglichkeiten des erfindungsgemäßen Apparates und Verfahrens. Beispielhaft seien aufgezählt:
- Feststellung einer zeitlichen Veränderung einer Probe, z. B. vor und nach einem operativen Eingriff oder während eines Heilungsprozesses. Die Reproduzierbarkeit der Intensitätsprofile ermöglicht Langzeituntersuchungen, z. B. im Rahmen von krankhaften Veränderungen der Haut oder der Kontrolle der Größe von Hautveränderungen (Leberflecke, Muttermale). Daneben eignet sich das Verfahren zur Untersuchung von eingeschlossenen Fremdkörpern in der Haut.
- Klassifizierung von verschiedenen Hauttypen. Für diesen Fall ist die Verwendung von Diskriminanzanalysen zur Erstellung der Typen und Zuordnung einzelner Proben zu diesen Typen vorteilhaft.
   Insbesondere durch die gute Ortsauflösung in Bewegungsrichtung können aus Intensitäts-Weg Profilen durch mathematische Behandlung Größen abgeleitet werden, womit bestimmte Hautpartien erfolgreich identifiziert werden können- Beispielsweise können durch Fouriertransformation des Intensitätsprofils für einzelne Gewebstypen und eine anschließende Diskriminanzanalyse charakteristische periodisch wiederkehrende Eigenschaften der Gewebstypen ermittelt werden.
- Erfassung örtlich oder zeitlich homogener Stellen. Gewebe, insbesondere menschliche Haut, ist örtlich inhomogen durch darin oder darunter liegende Strukturen. Manche Körperteile sind fiir diagnostische Zwecke mehr oder weniger gut geeignet. Insbesondere, wenn eine nicht-invasive Erfassung von Analytkonzentrationen in bestimmten Gewebeteilen, z. B. mittels einer nicht-invasiven Ermittlung der Glukosekonzentration, gemäß PCT-DE 93/01058 oder US-A-5,028,787, vorgenommen werden soll, gibt es fiir eine Messung besser oder schlechter geeignete Orte. Mit Hilfe der vorliegenden Erfindung können gut geeignete Meßstellen festgestellt und markiert werden. Dies ist auch von Vorteil, wenn das nicht-invasive Meßgerät zwischen zwei Messungen von der Probe entfernt und später wieder befestigt werden soll. Das erfindungsgemäße Verfahren ist auch zur Erfassung der Größe und/oder zeitlichen Änderung homogener Bereiche der Probe geeignet
- Die geschilderten Methoden lassen sich sinngemäß auch auf nicht biologische Proben übertragen. So ermöglicht der erfindungsgemäße Apparat eine zerstörungsfreie Materialprüfung in streuenden Medien. Dies schließt beispielsweise Homogenitätsprüfungen bei Kunststoffen sowie Kontrolle von Formteilen mit Strukturen unterhalb der Oberfläche ein.

### Bezugszeichenliste

- 1: Erfindungsgemäße Anordnung
- 2: Lichtquellen
- 3: Detektoren
- 4: Walzen
- 5: Bewegungsrichtung
- 10: Grafische und/oder numerische Darstellung der Lichtintensität als Funktion des Ortes
- 11: Zeitgeber/Taktgeber
- 12: Elektronische Auswerteeinheit, Verrechnung von Weg und Lichtintensität
- 13: Lichtquellen und Ansteuerungselektronik
- 14: Detektoren und Verstärkungselektronik
- 15: Wegaufnahme

## Patentansprüche

1. Apparat zur optischen Charakterisierung von innerer Struktur oder/und Zusammensetzung einer ausgedehnten streuenden Probe durch Bewegung des Apparates auf einer Oberfläche der Probe, beinhaltend
a) eine Anordnung von einer oder mehreren Lichtquellen und einem oder mehreren Lichtdetektoren, bei der sich die eine oder die mehreren Lichtquellen und der eine oder die mehreren Lichtdetektoren an der gleichen Grenzfläche der Probe befinden,
b) einen Wegaufnehmer, der bei Bewegung des Apparates auf der Grenzfläche der Probe den zurückgelegten Weg festhält sowie
c) einen Auswerteteil, in dem die an einer bestimmten Stelle der Probe in dem einen oder den mehreren Lichtdetektoren empfangenen Signale mit der über den Wegaufnehmer erhaltenen relativen Lage gegenüber dem Apparat korreliert wird.

2. Apparat gemäß Anspruch 1, dadurch gekennzeichnet, daß er mehrere Lichtquellen und mehrere Lichtdetektoren aufweist.

3. Apparat gemäß Anspruch 2, dadurch gekennzeichnet, daß die Lichtquellen und Lichtdetektoren paarweise von jeweils einer Lichtquelle und einem Lichtdetektor ausgewertet werden.

4. Apparat gemäß Anspruch 3, dadurch gekennzeichnet, daß Lichtquelle und Lichtdetektor jeden Paares jeweils den gleichen Abstand d voneinander haben.

5. Apparat gemäß Anspruch 4, dadurch gekennzeichnet, daß der Abstand d zwischen Lichtquelle und Lichtdetektor zwischen 0.5 mm und 100 mm liegt.

6. Apparat gemäß Anspruch 1, dadurch gekennzeichnet, daß die Detektoren zur Bestimmung der Lichtintensität, des Polarisationsgrades oder der Laufzeit von Photonen geeignet sind.

7. Verfahren zur optischen Charakterisierung von innerer Struktur und/oder Zusammensetzung einer ausgedehnten streuenden Probe durch Bewegung eines Apparates auf einer Grenzfläche der Probe mit einem Apparat beinhaltend,
a) eine Anordnung von einer oder mehreren Lichtquellen und einem oder mehreren Lichtdetektoren, bei der sich die eine oder die mehreren Lichtquellen und der eine oder die mehreren Lichtdetektoren bei der optischen Charakterisierung der Probe an der gleichen Grenzfläche der Probe befinden,
b) einen Wegaufnehmer sowie
c) einen Auswerteteil, wobei
der Apparat auf einer Grenzfläche der Probe bewegt wird und mit dem Wegaufnehmer die Bewegung des Apparates festgehalten wird,
die eine oder die mehreren Lichtquellen Licht aussenden, diffuses Sekundärlicht mit dem einen oder den mehreren Lichtdetektoren empfangen wird und
mit dem Auswerteteil die an einer bestimmten Stelle der Probe empfangenen Signale des einen oder der mehreren Lichtdetektoren mit der relativen Lage des Apparates korreliert werden.

8. Verfahren gemäß Anspruch 7, dadurch gekennzeichnet, daß während der Bewegung des Apparates das erhaltene Signal in Funktion von der zurückgelegten Wegstrecke gespeichert wird.

9. Verfahren gemäß Anspruch 7, dadurch gekennzeichnet, daß die Leuchtzeit der einzelnen Lichtquellen getaktet ist.

10. Verfahren gemäß Anspruch 7, dadurch gekennzeichnet, daß die Leuchtzeiten unterschiedlicher Lichtquellen zeitlich versetzt getaktet sind.

11. Verfahren gemäß Anspruch 7, dadurch gekennzeichnet, daß aus den gemessenen Signalen und der zurückgelegten Wegstrecke ein 2-dimensionales Intensitätsprofil erzeugt wird.

12. Verfahren gemäß Anspruch 7, dadurch gekennzeichnet, daß die Messung des Lichtes in Reflektion geschieht.

13. Verfahren gemäß Anspruch 7, dadurch gekennzeichnet, daß die Lichtquelle Licht einer Wellenlänge zwischen 400 und 10000 nm ausstrahlt.

14. Verfahren gemäß Anspruch 7, dadurch gekennzeichnet, daß der Abstand jedes Paares aus Lichtquelle und Lichtdetektor kleiner als bzw. nur geringfügig größer ist als die zu messende Inhomogenität.

15. Verfahren gemäß Anspruch 7 zur Erfassung einer zeitlichen Änderung der Struktur oder/und Zusammensetzung.

16. Verfahren gemäß Anspruch 7 zur Klassifizierung von Hauttypen.

17. Verfahren gemäß Anspruch 7 zur Erfassung örtlich oder zeitlich homogener Stellen.

18. Verfahren gemäß Anspruch 7 zur Erstellung von Hauttypenklassen über periodisch wiederkehrende Struktureigenschaften der Haut.

19. Verfahren gemäß Anspruch 7 zur Materialprüfungin technischen Formkörpern.

## Claims

1. Apparatus for the optical characterization of the internal structure and/or composition of a spatially extended, scattering sample by moving the apparatus on the surface of the sample, comprising
a. an arrangement of one or several light sources and one or several light detectors, where the one or several light sources and the one or several light detectors are located on the same surface of the sample.
b. a displacement sensor to record the distance that is covered by the movement of the apparatus on the surface of the sample, and
c. an evaluation unit in which the signals received by the one or several light detectors from the light source at a given site of the sample are correlated with the relative location provided by the displacement sensor with respect to the apparatus

2. Apparatus according to claim 1, characterized in that it comprises several light sources and several light detectors.

3. Apparatus according to claim 2, characterized in that the light sources and the light detectors are evaluated in pairs consisting of one light source and one light detector.

4. Apparatus according to claim 3, characterized in that light source and the light detector of each pair are spaced apart from each other at an identical distance d.

5. Apparatus according to claim 4, characterized in that the distance d between light source and the light detector is between 0.5 and 100 mm.

6. Apparatus according to claim 1, characterized in that the detectors are suitable for determining the light intensity, the degree of polarization, or the travel time of photons.

7. Method for the optical characterization of the internal structure and/or composition of a spatially extended, scattering sample by moving an apparatus on the surface of a sample with the apparatus comprising
a. an arrangement of one or several light sources and one or several light detectors, where, during the optical characterization of the sample, the one or several light sources and the one or several light detectors are located on the same surface of the sample.
b. a displacement sensor, and
c. an evaluation unit
where the apparatus is moved on the surface of the sample while the displacement sensor records said movement of the apparatus,
where the one or several light sources emit lignt, and where diffuse secondary light is received by the one or several light detectors, and
in which the evaluation unit correlates the signals received by the one or several light detectors from the light source at a given site of the sample with the relative location of the apparatus.

8. Method according to claim 7, characterized in that during movement of the apparatus, the generated signal is stored as a function of the path covered.

9. Method according to claim 7, characterized in that the activation cycle of the individual light sources is timed.

10. Method according to claim 7, characterized in that the activation cycles of different light sources are timed in different cycles.

11. Method according to claim 7, characterized in that a two-dimensional intensity profile is generated based on the measured signals and the covered path.

12. Method according to claim 7, characterized in that the light is measured in terms of reflection.

13. Method according to claim 7, characterized in that the light source emits light of a wavelength between 400 and 10 000 nm.

14. Method according to claim 7, characterized in that the distance between light source and light detector of each pair is smaller or only slightly larger than the inhomogeneity to be measured.

15. Method according to claim 7 to sense time-related alterations to the structure or composition involved.

16. Method according to claim 7 to classify skin types.

17. Method according to claim 7 to sense the homogeneity of sites in a local or time-related manner.

18. Method according to claim 7 to establish classes of skin types over periodically repeated structural properties of the skin.

19. Method according to claim 7 for material testing in formed pieces.

## Revendications

1. Appareil pour caractériser optiquement une structure ou/et composition interne d'un prélèvement dispersif étendu en déplaçant l'appareil sur une surface du prélèvement, contenant
a) une disposition d'une ou plusieurs sources lumineuses et d'un ou plusieurs détecteurs de lumière, dans lequel lesdites une ou plusieurs sources lumineuses et lesdits un ou plusieurs détecteurs de lumière se trouvent au niveau de la même surface limite du prélèvement,
b) un capteur de déplacement qui mémorise le chemin parcouru lors du déplacement de l'appareil sur la surface limite, et
c) un élément d'analyse, dans lequel les signaux reçus au niveau d'une position déterminée du prélèvement dans lesdits un ou plusieurs détecteurs de lumière sont corrélés avec l'emplacement obtenu par le capteur de déplacement.

2. Appareil selcn la revendication 1, caractérisé en ce qu'il comporte plusieurs sources de lumière et plusieurs détecteurs de lumière.

3. Appareil selon la revendication 2, caractérisé en ce que les sources de lumière et détecteurs de lumière sont exploités par paires, respectivement une source de lumière et un détecteur de lumière.

4. Appareil selon la revendication 3, caractérisé en ce que la source de lumière et le détecteur de lumière de chaque paire sont séparés de la même distance d.

5. Appareil selon la revendication 4, caractérisé en ce que la distance d entre source de lumière et détecteur de lumière est comprise entre 0,5 mm et 100 mm.

6. Appareil selon la revendication 1, caractérisé en ce que, les détecteurs de lumière sont adaptés à la détemination de l'intensité lumineuse, du degré de polarisation et du temps de propagation des photons.

7. Procédé pour caractériser optiquement une structure ou/et composition interne d'un prélèvement dispersif étendu en déplaçant un appareil sur une surface du prélèvement, avec un appareil contenant,
a) une disposition d'une ou plusieurs sources lumineuses et d'un ou plusieurs détecteurs de lumière, dans lequel lesdites une ou plusieurs sources lumineuses et lesdits un ou plusieurs détecteurs de lumière se trouvent au niveau de la même surface limite du prélèvement lors de la caractérisation optique du prélèvement,
(b) un capteur de déplacement, et
(c) un élément d'analyse, dans lequel
l'appareil est déplacé sur une surface limite du prélèvement et le déplacement de l'appareil est mémorisé à l'aide du capteur de déplacement,
lesdites une ou plusieurs sources de lumière émettent de la lumière, une lumière secondaire diffuse est reçue avec lesdits un ou plusieurs détecteurs de lumière et
à l'aide de l'élément d'analyse, les signaux reçus au niveau d'une position déterminée du prélèvement, provenant desdits un ou plusieurs détecteurs de lumière, sont corrélés avec l'emplacement relatif de l'appareil.

8. Procédé selon la revendication 7, caractérisé en ce que, pendant le déplacement de l'appareil, le signal obtenu est mémorisé en fonction de la distance parcourue.

9. Procédé selon la revendication 7, caractérisé en ce que, la durée d'illumination des sources de lumière individuelles est synchronisée.

10. Procédé selon la revendication 7, caractérisé en ce que, les durées d'illumination des différentes sources de lumière individuelles sont synchronisées et décalées dans le temps.

11. Procédé selon la revendication 7, caractérisé en ce que, un profil d'intensité bidimensionnel est produit à partir des signaux mesurés et dudit chemin parcouru.

12. Procédé selon la revendication 7, caractérisé en ce que, la mesure de la lumière a lieu par réflexion.

13. Procédé selon la revendication 7, caractérisé en ce que, la source lumineuse émet de la lumière à une longueur d'onde comprise entre 400 et 10000 nm.

14. Procédé selon la revendication 7, caractérisé en ce que, la distance séparant chaque paire de source lumineuse et détecteur de lumière est plus petite ou seulement à peine plus grande que la taille de l'inhomogénéité à mesurer.

15. Procédé selon la revendication 7 pour détecter une modification temporelle de ladite structure ou/et composition.

16. Procédé selon la revendication 7 pour classifier les types de peau.

17. Procédé selon la revendication 7 pour détecter spatialement ou temporellement des positions homogènes.

18. Procédé selon la revendication 7 pour établir des classes de types de peau à l'aide des propriétés structurelles se répétant périodiquement de la peau.

19. Procédé selon la revendication 7 pour le contrôle de matériaux dans des corps façonnés de la technique.
